# EUROPEAN PATENT SPECIFICATION

(11) **EP 3 941 476 B1**
(45) Date of publication and mention of the grant of the patent: **31.05.2023**
(21) Application number: 20714965.9
(22) Date of filing: 20.03.2020
(51) Int. Cl.: A61K 31/52, A61P 1/16

(54) **ROSCOVITINE ANALOGUES AND USE THEREOF FOR TREATING RARE BILIARY DISEASES**
ROSCOVITINANALOGA UND VERWENDUNG DAVON ZUR BEHANDLUNG SELTENER GALLENERKRANKUNGEN
ANALOGUES DE ROSCOVITINE ET LEUR UTILISATION POUR LE TRAITEMENT DE MALADIES BILIAIRES RARES

(30) Priority: 20.03.2019 EP 19305340
(43) Date of publication of application: 26.01.2022
(73) Proprietor: SORBONNE UNIVERSITE, 75006 Paris (FR); Institut National de la Santé et de la Recherche Médicale (INSERM), 75654 Paris Cedex 13 (FR); Assistance Publique-Hôpitaux de Paris (APHP), 75004 Paris (FR); Manros Therapeutics, 29680 Roscoff (FR)
(72) Inventor: FALGUIERES, Thomas, 91260 Juvisy-sur-Orge (FR); VAUTHIER, Virginie, 91430 Igny (FR); MEIJER, Laurent, 29680 Roscoff (FR); OUMATA, Nassima, 29680 Roscoff (FR)
(74) Representative: Icosa
(86) International application number: PCT/EP2020/057833
(87) International publication number: WO 2020/188100

(56) References cited:
- EP-A1- 2 907 514
- NIKOLAY O. BUKANOV ET AL: "CDK inhibitors R-roscovitine and S-CR8 effectively block renal and hepatic cystogenesis in an orthologous model of ADPKD", CELL CYCLE, vol. 11, no. 21, 1 November 2012 (2012-11-01), pages 4040-4046, XP055617585, US ISSN: 1538-4101, DOI: 10.4161/cc.22375
- REICHERT MATTHIAS CHRISTIAN ET AL: "ABCB4 Gene Aberrations in Human Liver Disease: An Evolving Spectrum", SEMINARS IN LIVER DISEASE, THIEME MEDICAL PUBLISHERS, US, vol. 38, no. 4, 31 October 2018 (2018-10-31), pages 299-307, XP009515795, ISSN: 0272-8087, DOI: 10.1055/S-0038-1667299

## Description

### FIELD OF INVENTION

The present invention relates to purine derivative compounds, in particular roscovitine analogues. The present invention also relates to roscovitine analogues according to the invention for use as medicaments. Finally, the present invention relates to roscovitine analogues for their use for treating rare biliary diseases like intrahepatic cholestatic diseases, and more particularly ABCB4-related biliary diseases, characterized by a defect of phosphatidylcholine secretion in hepatocytes.

### BACKGROUND OF INVENTION

Rare biliary diseases like intrahepatic cholestatic diseases are hereditary pathologies that both affect children's and adult's liver performance by inducing progressive liver disease, which typically leads to liver failure. Indeed, liver cells are less able to secrete the bile thus decreasing intrahepatic bile flow, which favours the formation of a toxic bile and leads to liver failure in affected individuals. Generally, hepatic cell destruction may also be a consequence of intrahepatic biliary obstruction.

These dysfunctions affecting bile formation may be due to deficiency and abnormal functioning of membrane transporters. Among them, Adenosine triphosphate Binding Cassette transporter, subfamily B member 4 (ABCB4), also called MDR3, plays a great role in liver performance. This member of the ATP Binding Cassette (ABC) superfamily is mainly expressed at the canalicular membrane of hepatocytes where its function is to translocate phosphatidylcholine (PC) from the inner leaflet to the outer leaflet of the hepatocyte canalicular plasma membrane, thus allowing PC secretion into bile. At the molecular level, secreted PC has an essential role since it forms mixed micelles with the other co-secreted hydrophobic bile components, such as bile salts and cholesterol. Indeed, impairment of PC secretion into bile leads to the formation of cholesterol crystals and gallstones, as well as loss of protection from the detergent effects of free bile salts on biological membranes of the biliary tree.

These clinical characteristics have been largely reported for patients with genetic variations of the *ABCB4* gene (missense variations) which lead to rare biliary diseases, such as progressive familial intrahepatic cholestasis type 3 (PFIC3), low-phospholipid associated cholelithiasis (LPAC) syndrome or intrahepatic cholestasis of pregnancy (ICP). In the case of ABCB4-related biliary diseases, one of the reasons resulting in the impairment of PC secretion by ABCB4 transporter is the lack of cellular traffic preventing both its maturation and its plasma membrane localization. Therefore, this will induce both a retention of immature transporter in cell organelles responsible for protein's maturation and a deficiency in PC secretion.

For patients with ABCB4-related biliary diseases, the only pharmacological treatment is the administration of ursodeoxycholic acid (UDCA), a bile acid with low hydrophobicity. Although this therapy is efficient in the milder forms of the diseases, UDCA is not - or poorly - efficient in the majority of homozygous or compound heterozygous patients with severe forms of ABCB4-related diseases for whom the only alternative remains liver transplantation. In the absence of surgery and efficient pharmacological treatment able to cure patients, or at least delay liver transplantation, the life expectancy is low. Thus, pharmacological alternatives are eagerly needed.

One of the first alternatives considered is (R)-roscovitine, also known as Seliciclib or CYC202 and hereafter referred to as roscovitine. This 2,6,9-trisubstituted purine was identified as a relatively potent and selective Cyclin-Dependent Kinase (CDK) inhibitor. Roscovitine has undergone several studies in many indications up to clinical phase trials in various cancers, rheumatoid arthritis, glaucoma and cystic fibrosis. Interestingly, roscovitine has been shown to correct the intracellular localisation and the channel activity of CFTR, another ABC carrier family member involved in cystic fibrosis (EP2907514). Thus, roscovitine appeared as potential interesting treatment for ABCB4-related diseases. Moreover, studies show that treatment with 100µM of roscovitine lead to increase maturation and relocation of three an ER-retained ABCB4 variants (ABCB4-I541F / I490T / L556R); retained in the endoplasmic reticulum-ER as an immature and high-mannose glycosylated protein.

However, roscovitine displayed important dose-dependent cytotoxicity, which might be explained by its CDK inhibition activity, and inhibition of ABCB4-WT-mediated phosphatidylcholine secretion activity in HEK cells.

Therefore, there is still a need to develop compounds that shows the same activity that roscovitine but without cytotoxicity for the treatment of intrahepatic cholestatic diseases such as PFICs.

In order to overcome cytotoxicity, the Applicant develop an alternative strategy by synthesizing less toxic roscovitine structural analogues. These compounds were able to correct the intracellular traffic, thereby restoring the maturation, the canalicular expression and more importantly the function of ABCB4 variants (I541F / I490T / L556R).

### SUMMARY

The invention thus relates to Compound of formula (I): 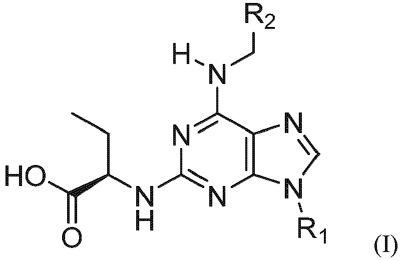 wherein
R₁ is
   - a (C₁-C₄) alkyl group, or
   - a (C₃-C₆) cycloalkyl group,
R₂ is
   - a phenyl or a substituted phenyl with one to three groups,
with the proviso that when R₂ is a phenyl then R₁ is a (C₃-C₆) cycloalkyl group,
or anyone of its pharmaceutically acceptable salt.

According to one embodiment, R₁ is a (C₁-C₄) alkyl group selected from methyl, ethyl, propyl, isopropyl. According to one embodiment, R₁ is isopropyl.

According to one embodiment, R₁ is cyclopentyl.

According to one embodiment, R₂ is a phenyl or substituted phenyl with one or two groups, said groups being selected from electron withdrawing groups and halogens. According to one embodiment, R₂ is a phenyl or a substituted phenyl with one or two groups, said groups being selected from CF₃, F, Cl and Br.

According to this embodiment, R₂ is a substituted phenyl with one to three groups and at least one group being in *meta* position. According to this embodiment, R₂ is a substituted phenyl with one to two groups and at least one group being in *meta* position.

According to one embodiment, R₁ is a cyclopentyl group and the group R₂ is selected from:

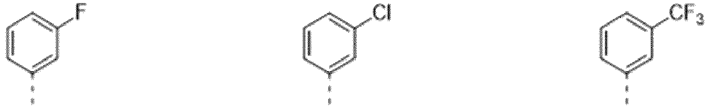

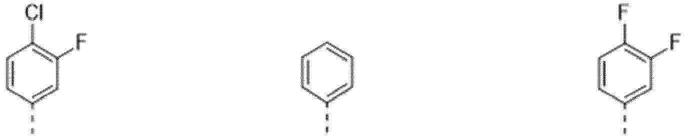

According to one embodiment, the compound of formula (I) is selected from:

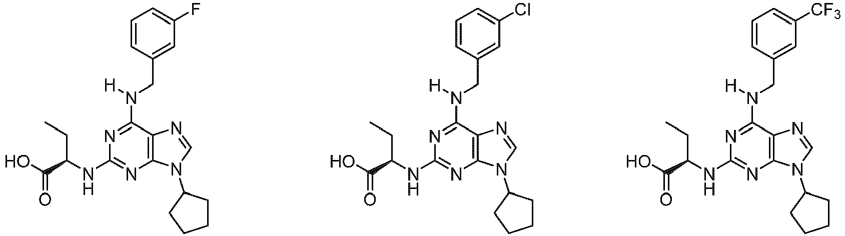

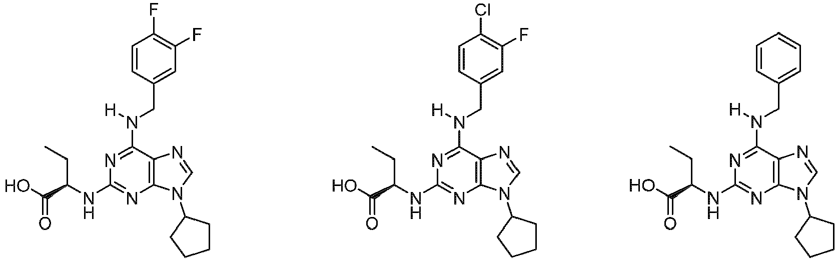

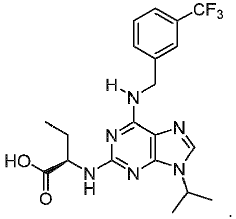

The invention further relates a composition comprising at least one compound of formula (I).

According to one embodiment, the composition is a pharmaceutical composition further comprising at least one pharmaceutically acceptable excipient.

The invention further relates to the compound of formula (I) for use as a medicament.

The invention further relates to the composition according to the invention for use as a medicament.

According to one embodiment, the invention relates to the compound of formula (I) or the composition according to the invention for use in the treatment of rare biliary diseases such as intrahepatic cholestatic diseases.

According to another embodiment, the invention relates to the compound of formula (I) or the composition according to the invention for use in the treatment of intrahepatic cholestatic diseases being an ABCB4-related biliary disease, characterized by a defect of phosphatidylcholine secretion in hepatocytes.

According to another embodiment, the invention relates to the compound of formula (I) or the composition according to the invention for use in the treatment of progressive familial intrahepatic cholestasis (PFIC3), low phospholipid-associated cholelithiasis (LPAC) syndrome, intrahepatic cholestasis of pregnancy (ICP), drug-induced liver injury, transient neonatal cholestasis (TNC), adult biliary fibrosis and cirrhosis or intrahepatic cholangiocarcinoma (IHCC).

According to another embodiment, the invention relates to the compound of formula (I) or the composition according to the invention for use in the treatment of intrahepatic cholestatic diseases that are selected from the group consisting of: progressive familial intrahepatic cholestasis type 3, LPAC syndrome or ICP.

### DEFINITIONS

In the present invention, the following terms have the following meanings:
- **"Phenyl"** refers to a cyclic aromatic group of atoms with the formula C₆H₅.
- **"Substituted phenyl"** refers to a cyclic aromatic group of atoms with the formula (R)ₙ-C₆H₅, wherein R is a substituent and n is comprised between 1 and 5, R being the same or different. In one embodiment, n is equal to 1. In one embodiment, n is equal to 2. In one embodiment, n is equal to 3. In one embodiment, R is in *meta* position of the phenyl group. In one embodiment, R is in *para* position of the phenyl group. In one embodiment, n is equal to 2, and R is in the *para* and *meta* position of the phenyl group.
- **"Halogen"** refers to chlorine, fluorine, bromine, or iodine, and in particular denotes chlorine, fluorine, bromine.
- "**(C₁-C₄) alkyl group"** refers to a C₁-C₄ linear- or branched- saturated hydrocarbon chain. Examples are, but not limited to, methyl, ethyl, propyl, isopropyl.
- "**(C₃-C₆) cycloalkyl group"** refers to a C₃-C₆ cyclic saturated hydrocarbon. Examples are, but not limited to, cyclobutyl, cyclopentyl, cyclohexane.
- **"Electron withdrawing group"** refers to a functional group having the ability to attract electrons. Examples are, but not limited to, CN, NO₂, CF₃.
- **"Endoplasmic reticulum** - **ER"** refers to a continuous membrane system that forms a series of flattened sacs within the cytoplasm of eukaryotic cells and serves multiple functions, being important particularly in the synthesis, folding, modification, and transport of proteins. All eukaryotic cells contain an endoplasmic reticulum (ER).
- **"Wild Type (WT)"** refers to a phenotype, genotype, or gene that predominates in a natural population of organisms or strain of organisms in contrast to that of natural or laboratory mutant forms.
- **"ABCB4-WT"** as used herein refers to the phenotype of the typical form of the ABCB4 transporter as it occurs in nature.
- **"ABCB4 variants"** as used herein refers to the abnormal phenotype of the mutated form of the ABCB4 transporter. For examples, but not limited to, ABCB4-I541F, ABCB4-I490T, ABCB4-L556R.
- **"ER-retained ABCB4 variants"** refers to ABCB4 variants retained in the ER as an immature protein and thus cannot be correctly addressed at the plasma membrane in order to fulfil their physiological functions.
- **"Homozygous"** having the two genes at corresponding loci (or position) on homologous chromosomes identical for one or more loci.
- **"Compound heterozygous"** having the two alleles at corresponding loci on homologous chromosomes different for one or more loci.
- **"Treatment"** as used herein, refers to both therapeutic and prophylactic (or preventive) measures, whose object is to prevent or slow down (lessen) the targeted pathologic condition or disorder. Those in need of treatment include those already with the disorder as well as those prone to have the disorder or those in whom the disorder is to be prevented. A subject or mammal is successfully "treated" if, after receiving a therapeutic amount of the compound or composition according to the present invention, the patient shows one or more of the following observable and/or measurable changes: amelioration related to one or more of the symptoms associated with the specific disease or condition, reduction of morbidity and mortality and improvement in quality of life issues. The above parameters for assessing successful treatment and improvement in the disease are readily measurable by routine procedures familiar to a physician.
- **"Therapeutically effective amount"** as used herein, refers to the level or amount of the compound or composition according to the present invention, that is aimed at (but without causing significant negative or adverse side effects to the subject): (1) delaying or preventing the onset of the targeted condition or disorder; (2) slowing down or stopping the progression, aggravation, or deterioration of one or more symptoms of the targeted condition or disorder; (3) bringing about ameliorations of the symptoms of the targeted condition or disorder; (4) reducing the severity or incidence of the targeted condition or disorder; and/or (5) curing the targeted condition or disorder. A therapeutically effective amount of the compound or composition according to the present invention may be administered prior to the onset of the targeted condition or disorder, for a prophylactic or preventive action.
- **"Pharmaceutically acceptable excipient"** refers to an excipient that does not produce an adverse, allergic or other untoward reaction when administered to an animal, preferably a human. It includes any and all dispersion media and solvents, coatings, isotonic and absorption delaying agents, additives, preservatives, stabilizers and the like. For human administration, preparations should meet sterility, pyrogenicity, general safety and purity standards as required by regulatory offices, such as, for example, FDA Office or EMA.
- **"Subject"** as used herein, refers to a warm-blooded animal, preferably a human, a pet or livestock. As used herein, the terms **"pet"** and **"livestock"** include, but are not limited to, dogs, cats, guinea pigs, rabbits, pigs, cattle, sheep, goats, horses and poultry. In some embodiments, the subject is a male or female subject. In some embodiments, the subject is an adult (for example, a subject above the age of 18 (in human years) or a subject after reproductive capacity has been attained). In some embodiments, the subject may be a **"patient",** *i.e*., a subject who/which is awaiting the receipt of or is receiving medical care or was/is/will be the object of a medical procedure according to the methods of the present invention or is monitored for the development of a disease.

### DETAILED DESCRIPTION

This invention relates to Compound of formula (I): 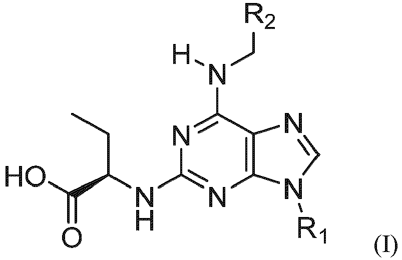 wherein
R₁ is
   - a (C₁-C₄) alkyl group, or
   - a (C₃-C₆) cycloalkyl group,
R₂ is
   - a phenyl or a substituted phenyl with one to three groups,
with the proviso that when R₂ is a phenyl then R₁ is a (C₃-C₆) cycloalkyl group,
or anyone of its pharmaceutically acceptable salt.

According to one embodiment, R₁ is a (C₁-C₄) alkyl group. According to one embodiment, R₁ is a (C₁-C₃) alkyl group. According to one embodiment, R₁ is selected from methyl, ethyl, propyl and isopropyl. According to one embodiment, R₁ is an isopropyl.

According to another embodiment, R₁ is a (C₃-C₆) cycloalkyl group. According to one embodiment, R₁ is a (C₄-C₆) cycloalkyl group. According to one embodiment, R₁ is a (C₅-C₆) cycloalkyl group. According to one embodiment when R₁ is selected from cyclopropyl, cyclobutyl, cyclopentyl and cyclohexane. According to one embodiment, R₁ is a cyclopentyl.

According to a one embodiment, R₂ is a phenyl group.

According to another embodiment, R₂ is a substituted phenyl with one to three groups. According to one embodiment, R₂ is a substituted phenyl with one to three groups selected from electron withdrawing group and halogens. According to one embodiment, R₂ is a substituted phenyl with one to three groups selected from CN, NO₂, CF₃, F, Cl, Br and I. According to one embodiment, R₂ is a substituted phenyl with one to three groups selected from CF₃, F, Cl and Br.

According to one embodiment, R₂ is a substituted phenyl with one to two groups selected from electron withdrawing group and halogens. According to one embodiment, R₂ is a substituted phenyl with one to two groups selected from CN, NO₂, CF₃, F, Cl, Br and I. According to one embodiment, R₂ is a substituted phenyl with one to two groups selected from CF₃, F, Cl and Br.

According to one embodiment, R₂ is a substituted phenyl with one group selected from electron withdrawing group and halogens. According to one embodiment, R₂ is a substituted phenyl with one group selected from CN, NO₂, CF₃, F, Cl, Br and I. According to one embodiment, R₂ is a substituted phenyl with one group selected from CF₃, F, Cl and Br.

According to this embodiment, when R₂ is a substituted phenyl with one to two groups, at least one of said group is in *meta* position of the phenyl ring.

In one embodiment, R₁ is a cyclopentyl group and the group R₂ is selected from:

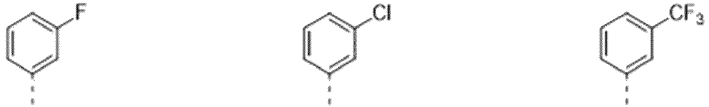

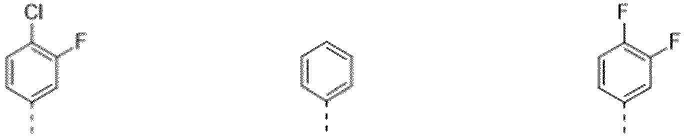

In one embodiment, R₁ is an isopropyl and R₂ is a 3-(trifluoromethyl)phenyl.

According to one embodiment, the compound of formula (I) is selected from:

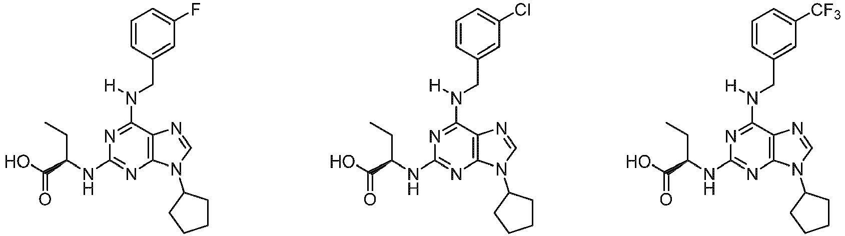

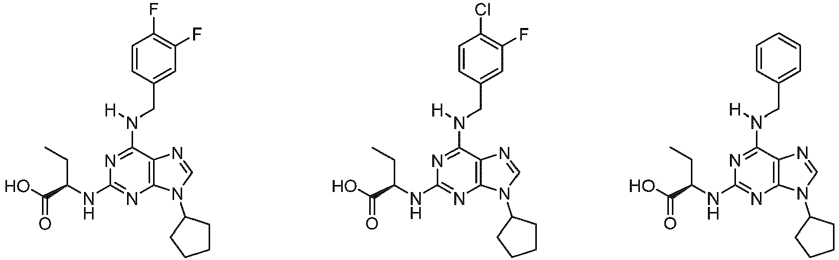

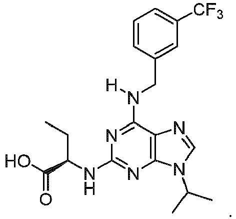

According to one embodiment, the compound of formula (I) is selected from:

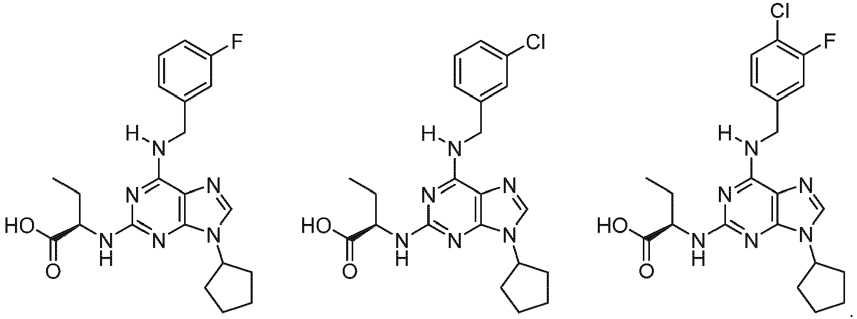

According to one embodiment, the compound of formula (I) is (*R*)-2-((9-cyclopentyl-6-((3-fluorobenzyl)amino)-9H-purin-2-yl)amino)butanoic acid:

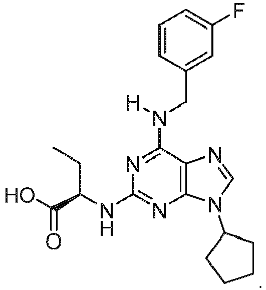

According to a one embodiment, the compound of formula (I) is (*R*)-2-((6-((3-chlorobenzyl)amino)-9-cyclopentyl-9H-purin-2-yl)amino)butanoic acid:

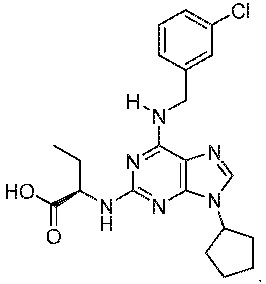

According to a further embodiment, the compound of formula (I) is (*R*)-2-((6-((4-chloro-3-fluorobenzyl)amino)-9-cyclopentyl-9H-purin-2-yl)amino)butanoic acid:

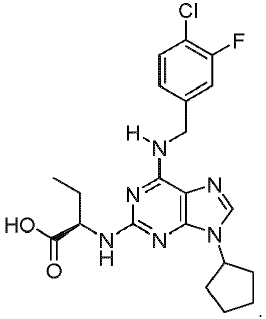

The compounds were named using ChemBioDraw^{®} Ultra version 12.0 (PerkinElmer).

The compounds of the invention may exist in the form of free bases or of addition salts with pharmaceutically acceptable acids. According to one embodiment, the compounds of the invention exist in the form of free bases. According to one embodiment, the compounds of the invention exist in the form of addition salts with pharmaceutically acceptable acids.

Suitable physiologically acceptable acid addition salts of compounds of formula (I) include hydrobromide, tartrate, citrate, trifluoroacetate, ascorbate, hydrochloride, triflate, maleate, mesylate, formate, acetate and fumarate.

The compounds of formula (I) and/or salts thereof may form solvates (e.g. hydrates) and the invention includes all such solvates.

The compound of the present invention can be prepared by conventional methods of organic chemistry practiced by those skilled in the art. In particular, the starting material may be 6-chloro-2-fluoropurine. One possible process is illustrated in example 1 hereinafter.

A further object of the invention is a medicament comprising, consisting or consisting essentially of at least one compound as described hereinabove.

Another object of the present invention is a composition comprising, consisting or consisting essentially of at least one compound as described hereinabove.

As used herein, **"consisting essentially of**", with reference to a composition, means that at least one compound according to the invention, or combination thereof is the only one therapeutic agent or agent with a biologic activity within said composition.

Another object of the invention is a composition wherein said composition is a pharmaceutical composition comprising, consisting or consisting essentially of at least one compound as described hereinabove and at least one pharmaceutically acceptable excipient.

Examples of pharmaceutically acceptable excipients include, but are not limited to, media, solvents, coatings, isotonic and absorption delaying agents, additives, stabilizers, preservatives, surfactants, substances which inhibit enzymatic degradation, alcohols, pH controlling agents, and propellants.

Examples of pharmaceutically acceptable media include, but are not limited to, water, phosphate buffered saline, normal saline or other physiologically buffered saline, or other solvent such as glycol, glycerol, and oil such as olive oil or an injectable organic ester. A pharmaceutically acceptable medium can also contain liposomes or micelles, and can contain immunostimulating complexes prepared by mixing polypeptide or peptide antigens with detergent and a glycoside.

Examples of isotonic agents include, but are not limited to, sugars, sodium chloride, and the like. Examples of agents that delay absorption include, but are not limited to, aluminum monostearate and gelatin.

Examples of additives include, but are not limited to, mannitol, dextran, sugar, glycine, lactose or polyvinylpyrrolidone or other additives such as antioxidants or inert gas, stabilizers or recombinant proteins (*e.g*., human serum albumin) suitable for *in vivo* administration.

Examples of suitable stabilizers include, but are not limited to, sucrose, gelatin, peptone, digested protein extracts such as NZ- Amine or NZ-Amine AS.

According to another aspect, the invention deals with the compound of formula (I) for use as a medicament.

According to another aspect, the invention deals with a composition comprising at least one compound of formula (I) for use as a medicament. In a more specific aspect, the composition is a pharmaceutical composition for use as a medicament comprising at least one pharmaceutically acceptable excipient.

The compound, the composition, the pharmaceutical composition or the medicament of the present invention may be administered orally, parenterally, by intraperitoneal administration, by inhalation spray, topically, rectally, nasally, buccally, vaginally or via an implanted reservoir.

In one embodiment, the compound, the composition, the pharmaceutical composition or the medicament of the present invention is injected. Examples of injections include, but are not limited to, intratumoral, intradermal, subcutaneous, intravenous, intramuscular, intra-lymphatic, intra-articular, intra-synovial, intrasternal, intrathecal, intravesical, intravaginal, intrahepatic, intralesional and intracranial injection or infusion techniques.

In a further aspect of the invention, the compound or the composition is administrated via subcutaneous, intradermal, intraperitoneal or intravaginal routes.

In one embodiment, the compound, the composition, the pharmaceutical composition or the medicament of the present invention is in a form adapted to oral administration. According to a first embodiment, the form adapted to oral administration is a solid form selected from the group comprising tablets, pills, capsules, soft gelatin capsules, sugarcoated pills, orodispersing tablets, effervescent tablets or other solids. According to a second embodiment, the form adapted to oral administration is a liquid form, such as, for example, a drinkable solution, a buccal spray, liposomal forms and the like.

A compound of formula (I) according to the invention may be formulated with excipients and components that are commonly used for oral compositions, as for example, fatty and/or aqueous components, humectants, thickeners, preserving agents, texture agents, taste agents and/or coating agents, antioxidants, preserving agents.

The formulating agents and excipients for an oral composition are known in this field and will not be the object of a fully detailed description herein. Many embodiments of oral compositions are formulated via usual processes for producing coated tablets, gel capsules, gels, controlled-release hydrogels, emulsions, tablets and capsules.

Examples of coating materials include, but are not limited to, lecithin.

In another embodiment, strain, the compound, the pharmaceutical composition or the medicament of the present invention is formulated for rectal or vaginal administration and may be presented as suppositories, pessaries, tampons, creams, gels, pastes, foams or sprays.

An example of formulation of a rectal composition, is a suppository, containing conventional suppository bases, such as cocoa butter or other glycerides. In another embodiment, strain, the compound, the pharmaceutical composition or the medicament of this invention is in a form suitable for parenteral administration. Forms suitable for parenteral administrations include, but are not limited to, sterile isotonic aqueous or nonaqueous solutions, dispersions, suspensions or emulsions, or sterile powders which may be reconstituted into sterile injectable solutions or dispersions just prior to use.

Parenteral administration comprises subcutaneous, intra-muscular and intra-venous administration. Formulations for injection may be presented in single-unit dosage form, such as ampoules or in multi-dose containers. The compositions may be formulated as suspensions, solutions or emulsions in oily or aqueous vehicles, and may contain additional agents, such as preservatives, emulsifying and/or stabilizing agents. Alternatively, a compound of formula (I) may be formulated as a dispersible powder, which may be prepared as a liquid composition, with a suitable vehicle, for example sterile water, just before use.

In another embodiment, the compound, the pharmaceutical composition or the medicament of the invention is in a form adapted for local delivery via the nasal and respiratory routes. Examples of formulations suitable for nasal or respiratory administration include, but are not limited to, nasal solutions, sprays, aerosols and inhalants.

In another embodiment, the compound, the pharmaceutical composition or the medicament of the invention is in a form adapted to a topical administration. Examples of formulations adapted to a topical administration include, but are not limited to, ointment, paste, eye drops, cream, patch, such as, for example, transdermal patch, gel, liposomal forms and the like.

In one embodiment, the composition or formulation of the invention may be presented in unit-dose or multi-dose sealed containers, for example, ampoules and vials, and may be stored in a lyophilized condition requiring only the addition of the sterile liquid excipient, for example water for injection, immediately prior to use. Extemporaneous injection solutions and suspensions may be prepared from sterile powders, granules and tablets.

A compound of formula (I) may be formulated as a liquid solution a liquid suspension or a powder.

As for liquid solution or suspension, a carrier may be typically pyrogen-free sterile or a dilute aqueous alcoholic solution. Liquid solution or suspension are preferably isotonic, hence may comprise sodium chloride. Optional additives include one or more preservative(s), such as for example methyl hydroxybenzoate, one or more antioxidant(s), one or more flavouring agent(s), one or more volatile oil(s), one or more buffering agent(s) and one or more surfactant(s).

As for a powder formulation, commonly used ingredients, such as a powdered diluent, for example powdered lactose, and surfactant(s) may be added.

Suitable formulations may also comprise one or more co-solvent(s), such as for example, ethanol, one or more surfactant(s), such as oleic acid and sorbitan trioleate, one or more antioxidant(s) and one or more suitable flavouring agent(s).

The exact dose for administration can be determined by the skilled practitioner, in light of factors related to the subject that requires treatment. Dosage is adjusted to provide sufficient levels of the composition or to maintain the desired effect of reducing signs or symptoms of the targeted pathologic condition or disorder, or reducing severity of the targeted pathologic condition or disorder. Factors which may be taken into account include the severity of the disease state (such as for example the tumor volume or the number of infected cells), the prognosis of the disease, the localization or accessibility to the tumor, general health of the subject, age, weight, and gender of the subject, diet, time and frequency of administration, drug combination(s), reaction sensitivities, and tolerance/response to therapy.

In one embodiment, a therapeutically effective amount of the strain, the composition, the pharmaceutical composition, the medicament or the vaccine composition of the present invention is administered (or is to be administered) to the subject.

A dosage regimen suitable for the administration of a compound of formula (I) falls within the technical skills of an artisan in the art, and depends from multiple parameters. Indeed, a suitable dosage regimen depends from the gender, the age, the weight, and the progress of the disease. Within the scope of the instant invention, a suitable dosage regimen may encompass about 1 to 500 mg of the active compound.

For example, for an oral administration, a drug may comprise about 1 to about 500 mg of active compound, for example about 20 to about 250 mg of active compound, for example about 50 to 150 mg of active compound.

The present invention further relates to the compound, the composition, the pharmaceutical composition or the medicament, for use in preventing and/or treating severe biliary disease in a subject in need thereof. It also relates to methods of preventing and/or treating severe biliary diseases, by administering to a subject in need thereof the compound, the composition, the pharmaceutical composition, or the medicament according to the present invention.

In one embodiment, the strain, the composition, the pharmaceutical composition, the medicament or the vaccine composition is for treating severe biliary diseases.

In one embodiment, the compound, the composition, the pharmaceutical composition or the medicament is for use in treating and/or preventing intrahepatic cholestatic diseases.

In one embodiment, the compound, the composition, the pharmaceutical composition or the medicament is for use in treating and/or preventing an ABCB4-related biliary disease.

In a still further embodiment, the compound, the composition, the pharmaceutical composition or the medicament is for use in treating and/or preventing intrahepatic cholestatic diseases, progressive familial intrahepatic cholestasis (PFIC3), low phospholipid-associated cholelithiasis (LPAC) syndrome, intrahepatic cholestasis of pregnancy (ICP), drug-induced liver injury, transient neonatal cholestasis (TNC), adult biliary fibrosis and cirrhosis or intrahepatic cholangiocarcinoma (IHCC).

In a still further embodiment, the compound, the composition, the pharmaceutical composition or the medicament is for use in treating and/or preventing progressive familial intrahepatic cholestasis type 3, LPAC syndrome or ICP.

In one embodiment, the subject is a human.

In one embodiment, the subject has a severe biliary disease. In one embodiment, the subject is diagnosed or has been diagnosed with a severe biliary disease.

In another embodiment, the pharmaceutical composition according to the present invention may be administered prior, during or after another pharmaceutical composition comprising an additional agent such as, but not limited to, ursodeoxycholic acid (UDCA).

In a further embodiment, the pharmaceutical composition according to the present invention may comprise an additional agent.

In one embodiment, the subject was not treated previously with another treatment for a severe biliary disease (*i.e*., the method of the invention is the first line treatment).

In another embodiment, the subject previously received one, two or more other treatments for a severe biliary disease for example with UDCA (*i.e*., the method of the invention is a second line, a third line or more). In one embodiment, the subject previously received one or more other treatments for a severe biliary disease, but was unresponsive or did not respond adequately to these treatments, which means that there is no or too low therapeutic benefit induced by these treatments.

In another embodiment, the subject is at risk of developing a severe biliary disease. Examples of risk factors for developing a severe biliary disease include, but are not limited to, family history of a severe biliary disease or genetic predisposition.

### BRIEF DESCRIPTION OF THE FIGURES

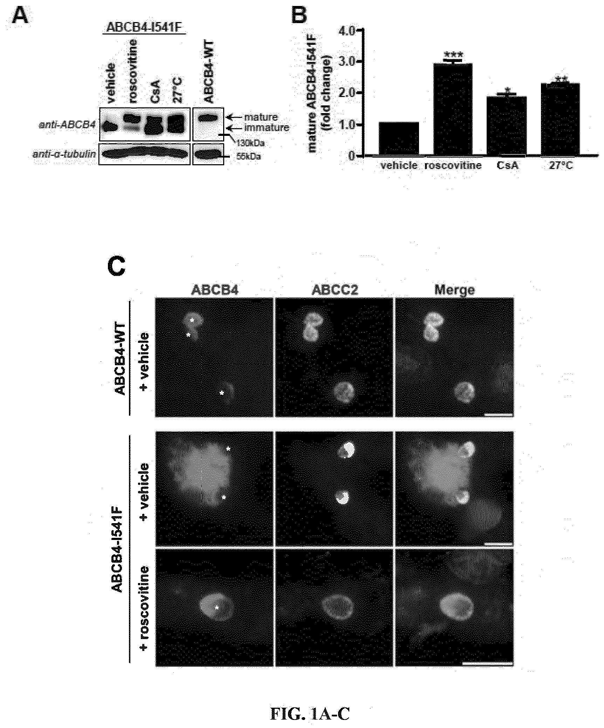

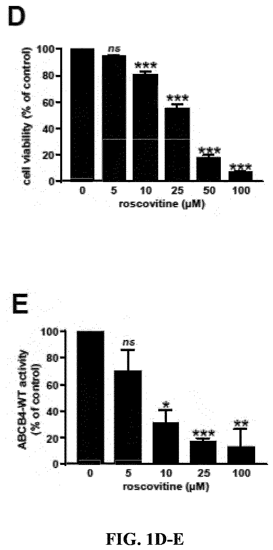

**Figure 1** indicates that roscovitine rescues the maturation and the traffic of ABCB4-I541F, but is cytotoxic and inhibitory of ABCB4 activity. **(A)** ABCB4-I541F was transiently expressed in HEK cells. After 16 hours of treatment with vehicle (DMSO), 100 µM roscovitine, 10 µM CsA or cell culture at 27°C, cell lysates were prepared and analysed by immunoblot using the indicated antibodies. ABCB4-WT expressed under the same conditions is shown as reference. The mature and immature forms of ABCB4 are indicated (arrows). This panel is representative of six independent experiments. **(B)** Densitometry analysis of A. The percentage of the mature form of ABCB4 was quantified and then expressed as fold change compared to vehicle-treated cells. Means (± SEM) of six independent experiments are shown. *P <0.05; **P <0.01; ***P <0.001. **(C)** ABCB4-WT or ABCB4-I541F were expressed in HepG2 cells. After 16 hours of treatment with vehicle (DMSO) or 100 µM roscovitine cells were fixed and permeabilized. After indirect immunofluorescence, ectopically expressed ABCB4 (left column) and endogenous ABCC2 (middle column) were visualized by fluorescence microscopy. Nuclei shown in the merged images were labelled with Hoechst 33342 (right column). Asterisks in the left panels indicate bile canaliculi. This panel is representative of three independent experiments. Bars: 10 µm. **(D)** HEK cells were treated with increasing concentrations of roscovitine during three days. Cell viability was assessed by MTT assay and expressed as a percentage of values for control vehicle-treated cells. Means (± SEM) of four independent experiments performed in triplicate are shown. ***P <0.001; ns: not significant. **(E)** After transient expression of ABCB4-WT, HEK cells were treated with increasing doses of roscovitine. Then the capacity of these cells to secrete PC was assessed and represented as a percentage of the activity for vehicle-treated control cells (expressing ABCB4-WT) after background subtraction. Means (± SEM) of at least three independent experiments performed in triplicate for each tested condition are shown. *P <0.05; **P <0.01; ^{∗∗∗}P <0.001; ns: not significant.

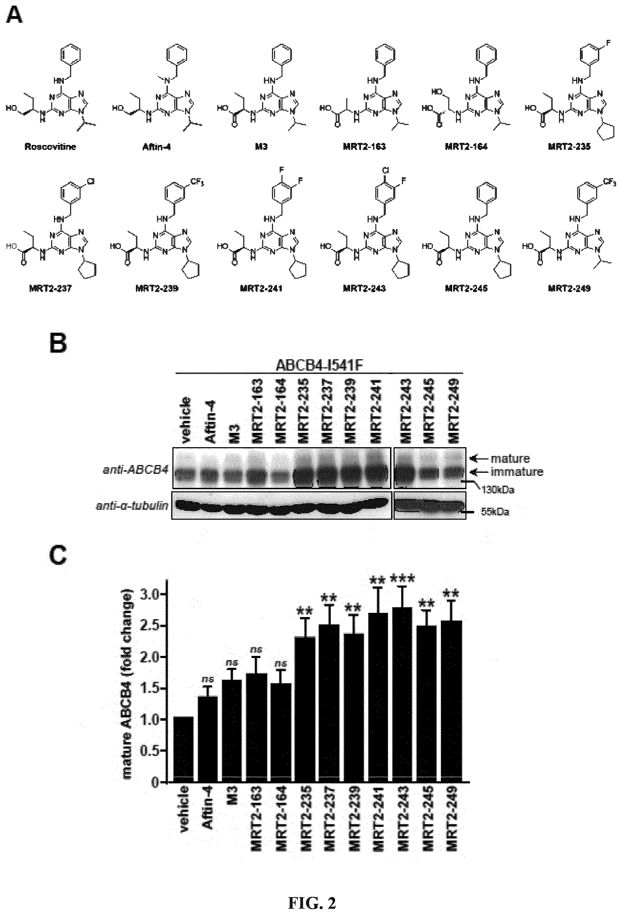

**Figure 2** shows that structural analogues of roscovitine correct the maturation of ABCB4-I541F. **(A)** Structure of roscovitine and its structural analogues. **(B)** After transient expression of ABCB4-I541F and treatment with 100 µM of the indicated compounds (or DMSO as vehicle), HEK cells were lysed and cell lysates were analysed by immunoblot using the indicated antibodies. The mature and immature forms of ABCB4 are shown. This panel is representative of five independent experiments. **(C)** Densitometry analysis of B. The percentages of the mature form of ABCB4 were quantified and then expressed as fold changes compared to the vehicle-treated condition. Means (± SEM) of five independent experiments are represented. **P <0.01; ^{∗∗∗}P <0.001; ns: not significant.

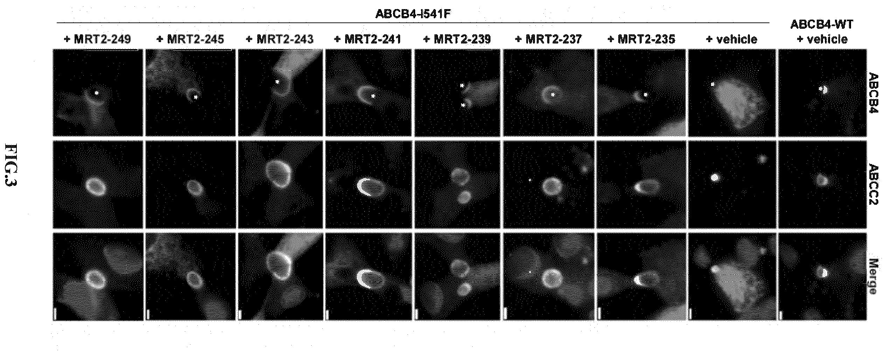

**Figure 3** shows that structural analogues of roscovitine restore canalicular targeting of ABCB4-I541F in HepG2 cells. After transient expression of ABCB4-WT or ABCB4-I541F and 16 hours of treatment with the vehicle (DMSO) as control or 100 µM of the indicated compounds, HepG2 cells were fixed and processed for indirect immunofluorescence and fluorescence microscopy to visualize ABCB4 (left panels) and ABCC2 (middle panels). Nuclei shown in the merge images were labelled with Hoechst 33342 (right panels). Asterisks in the left panels indicate bile canaliculi. This figure is representative of at least three independent experiments per condition. Bars: 5 µm.

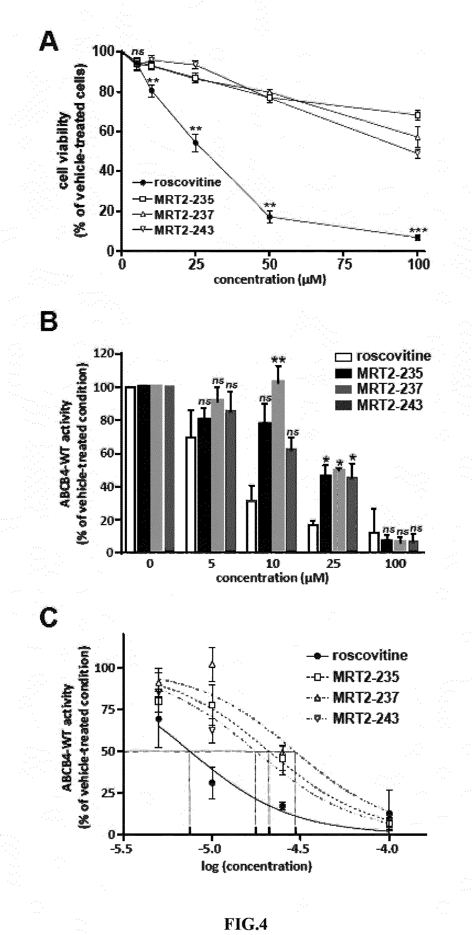

**Figure 4** shows that roscovitine analogues are less cytotoxic and less inhibitory of ABCB4 activity than roscovitine. **(A)** After treatment with increasing concentrations of the indicated compounds during three days, cell viability was determined and expressed as in Fig. 1D. Means (± SEM) of at least four independent experiments per condition performed in triplicate are shown. Statistics indicate comparisons between roscovitine treatment and the other conditions for each tested concentration: **P <0.01; ^{∗∗∗}P <0.001; ns: not significant. **(B)** After transient expression of ABCB4-WT, HEK cells were treated with increasing doses of roscovitine or its analogues as indicated. Then the capacity of these cells to secrete PC was assessed and expressed as in Fig. 1E. Means (± SEM) of at least three independent experiments performed in triplicate for each tested condition are shown. Statistics indicate comparisons between treatments with roscovitine and its analogues for each tested concentration: *P <0.05; **P <0.01; ns: not significant. **(C)** Results shown in B are represented as functions of log[concentration] for roscovitine and its analogues.

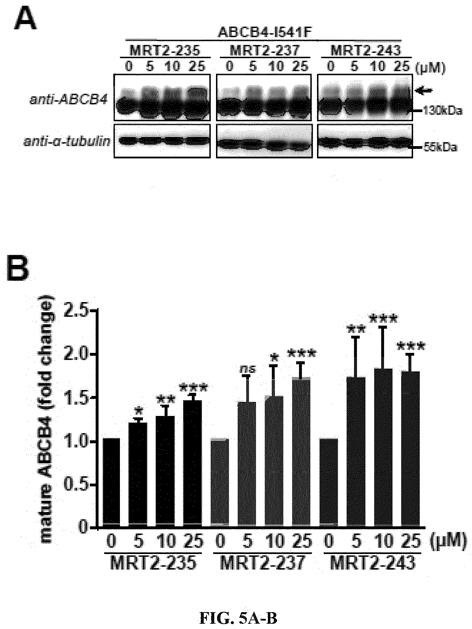

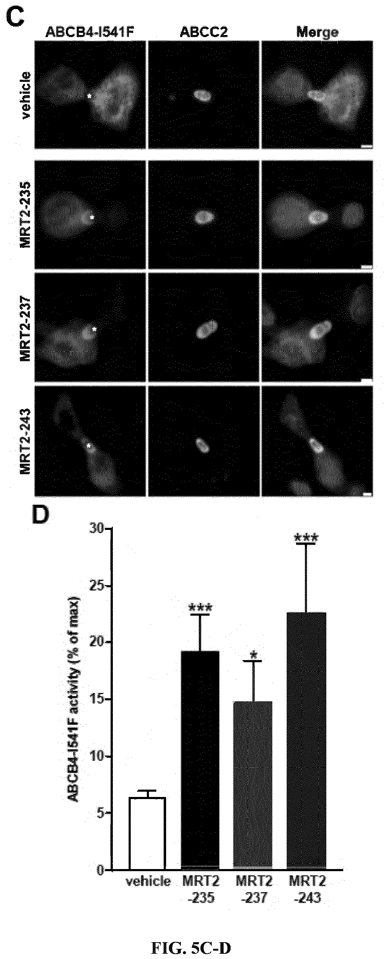

**Figure 5** shows that roscovitine analogues rescue the maturation, the plasma membrane targeting and the phosphatidylcholine secretion activity of ABCB4-I541F. **(A)** After transient expression of ABCB4-I541F in HEK cells and treatment with 0 (vehicle), 5, 10 or 25 µM of the indicated roscovitine analogues, cell lysates were prepared and analysed as in Fig. 2B. The arrow indicates the mature form of ABCB4. These immunoblots are representative of at least five independent experiments for each condition. **(B)** Densitometry analysis of A. The percentages of the mature form of ABCB4 were quantified and then expressed as fold changes compared to the vehicle-treated condition (0 µM) for each tested compound. Means (± SEM) of at least five independent experiments per roscovitine analogue are represented. *P <0.05; ^{∗∗}P <0.01; ^{∗∗∗}P <0.001; ns: not significant. **(C)** After transient expression of ABCB4-I541F, HepG2 cells were treated during 16 hours with 25 µM of the indicated roscovitine analogues. ABCB4 (left column) and ABCC2 (middle column) were immunolocalised as in Fig. 3. Nuclei are shown in the merge panels (right column). Asterisks in the left panels indicate bile canaliculi. This panel is representative of three independent experiments. Bars: 5 µm. **(D)** HEK cells expressing ABCB4-I541F were treated with 25 µM of roscovitine analogues, and ABCB4-mediated PC secretion was measured and represented as in Fig. 1E, the maximal activity being determined for cells expressing ABCB4-WT. Means (± SEM) of at least five independent experiments performed in triplicate for each tested condition are shown. *P <0.05; ^{∗∗∗}P <0.001.

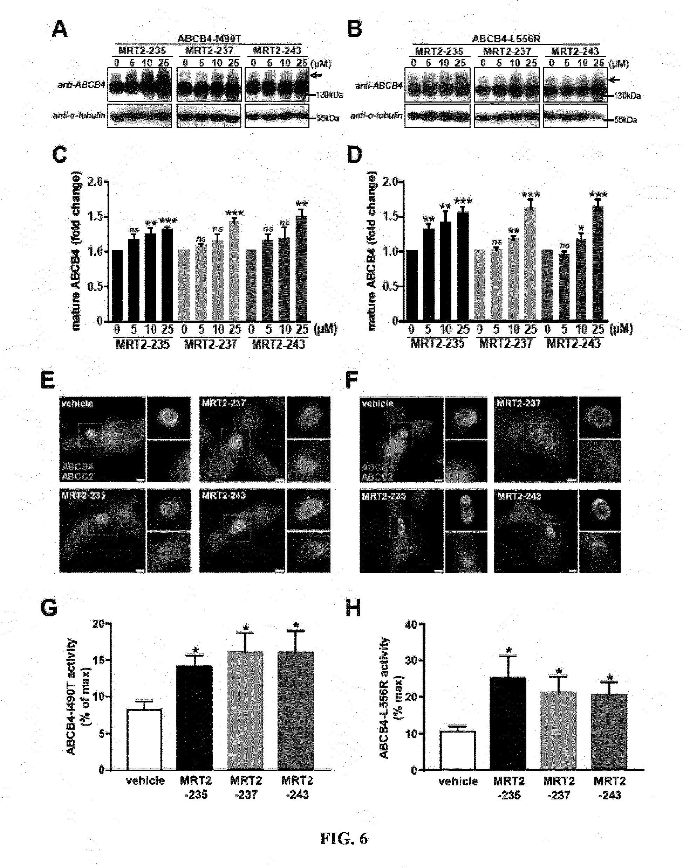

**Figure** 6 shows that roscovitine analogues are functional correctors of other ER-retained ABCB4 variants. **(A-B)** After treatment with 0 (vehicle), 5, 10 or 25 µM of the indicated roscovitine analogues, the maturation of ABCB4-I490T (A) and ABCB4-L556R (B) missense variants expressed in HEK cells was assessed by immunoblot as in Fig. 5A. These panels are representative of at least four independent experiments for each condition. **(C-D).** Densitometry analyses of A and B, respectively, as performed in Fig. 5B. Means (± SEM) of at least four independent experiments per condition are represented. *P <0.05; **P <0.01; ^{∗∗∗}P <0.001; ns: not significant. **(E-F).** After treatment without (vehicle) or with 25 µM of the indicated roscovitine analogues, HepG2 cells expressing ABCB4-I490T (E) or ABCB4-L556R (F) were processed for indirect immunofluorescence, as described in Fig. 5C. White squares indicate magnified areas in the individual frames shown on the right of each merged picture. Asterisks indicate bile canaliculi. Each panel is representative of three independent experiments. Bars: 5 µm. **(G-H).** ABCB4-mediated PC secretion of HEK cells expressing ABCB4-I490T (G) or ABCB4-L556R (H) and treated with 25 µM of the indicated roscovitine analogues was analysed as in Fig. 5D. Means (± SEM) of at least four independent experiments performed in triplicate for each tested condition are shown. *P <0.05.

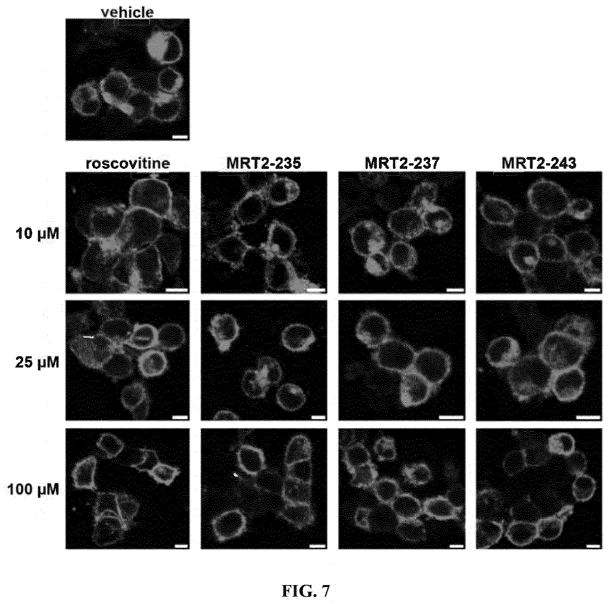

**Figure 7** shows the intracellular localization of ABCB4-WT in HEK cells. After transient expression of ABCB4-WT, HEK cells were treated with the indicated concentrations of roscovitine, MRT2-235, MRT2-237 or MRT-243 as in Fig. 4B. After fixation and permeabilization of the cells, localization of ABCB4-WT was assessed by indirect immunofluorescence and confocal microscopy. This figure is representative of three independent experiments. Bars: 5 µm.

### EXAMPLES

The present invention is further illustrated by the following examples.

### Example 1: Synthesis of compound 4 of formula (I):

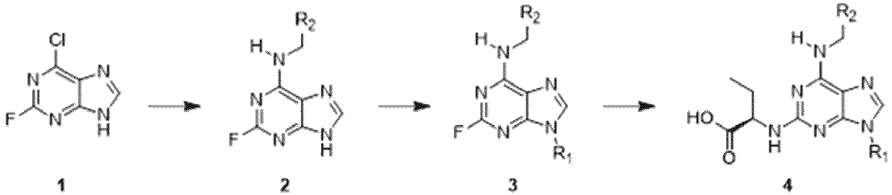

Compound **2** and compound **3** were prepared starting from 6-chloro-2-fluoropurine using a procedure starting from 2,6-dichloropurine (Oumata *et al.* 2009).

### Synthesis of compound 4

A mixture of compound 3 (1.7 mmol) and 3-aminobutanoic acid (12.26 mmol), K₃PO₄ (3.50 mmol) in 1 mL DMSO was heated at 160°C during 5h. After cooling to 20°C the mixture was diluted with 5 mL citric acid (10% in water, m:v). The mixture was extracted with EtOAc and the combined organic layers were washed with saturated NaCl and dried over Na2SO4. After evaporation of the solvent in vacuo, the crude product was purified on silica gel using CH₂Cl₂- EtOAc-THF (6:2:1).

### Example 2: Materials and Methods

### DNA constructs and mutagenesis

The subcloning of wild type (WT) ABCB4, isoform A (NM_000443.3), into pcDNA3 vector has been described (6). The I541F, I490T and L556R missense ABCB4 variants were previously reported and described (2, 6, 11). Site directed mutagenesis were performed using the QuikChange II XL site-directed mutagenesis kit (Agilent Technologies, Les Ulis, France), following manufacturer's instructions, and using the primers (Eurogentec, Angers, France) described in Table 1 below. The sequences of all constructs were systematically verified by automated sequencing.

**Table 1: Primers used for ABCB4 mutagenesis**

| Variant | Sequence (5'>"3) | |
|---|---|---|
| I490T | Sense | GTTTTCCACCACAATTGCTGAAAATACTTGTTATGGCCGTG (SEQ ID No 1) |
| | Antisense | CACGGCCATAACAAGTATTTTCAGCAATTGTGGTGGAAAAC (SEQ ID No 2) |
| I541T | Sense | GGTGGGCAGAAGCAGAGGTTCGCCATTGCACGTGCC (SEQ ID No 3) |
| | Antisense | GGCACGTGCAATGGCGAACCTCTGCTTCTGCCCACC (SEQ ID No 4) |
| L556R | Sense | CAAGATCCTTCTGCGGGATGAGGCCAC (SEQ ID No 5) |
| | Antisense | GTGGCCTCATCCCGCAGAAGGATCTTG (SEQ ID No 6) |

### Cell culture and transfection

Human embryonic kidney (HEK-293, herein referred to as HEK; ATCC^{®}-CRL-1573TM) cells and human hepatocellular carcinoma HepG2 (ATCC^{®}- HB-8065TM) cells were obtained from ATCC (Manassas, VA). As we previously reported, both HEK and HepG2 cells do not express ABCB4 (2, 12). Cells were grown in an incubator at 37°C with 5% CO2 in Dulbecco's Modified Eagle Medium (Gibco-Thermo Fisher Scientific, Villebon-sur-Yvette, France) containing 4.5 g/L D-glucose and supplemented with 10% heat-inactivated fetal bovine serum (Sigma, Saint-Quentin Fallavier, France), 2 mM L-glutamine, 2 mM sodium pyruvate, 100 units/mL of penicillin and 100 µg/mL streptomycin (Gibco-Thermo Fisher Scientific).

For transient transfection of HEK cells, they were seeded at subconfluent levels in the adequate tissue culture wells at least six hours before transfection. Turbofect (Thermo Fisher scientific) was used at a ratio of reagent:DNA of 2:1 according to manufacturer's instructions. For transient transfection of HepG2 cells, subconfluent cultures were seeded in the adequate culture wells 24 hours before transfection. Lipofectamine 3000 (Thermo Fisher Scientific) was used at a ratio of reagent:DNA of 1.5:1 according to manufacturer's instructions. Cell treatments and processing for further analyses were performed at least 16 hours post-transfection.

### Chemicals and cell treatments

Cyclosporin A (CsA) was from Santa Cruz Biotechnologies (Dallas, TX). (R)-roscovitine and its non-commercial structural analogues (Aftin-4, the metabolite M3, MRT2-163, MRT2-164, MRT2-235, MRT2-237, MRT2-239, MRT2-241, MRT2-243, MRT2-245, MRT2-248 and MRT2-249; see Fig. 2) were synthesized by ManRos Therapeutics (Roscoff, France). Each compound was solubilized in dimethylsulfoxide (DMSO) as 1000X concentrated stock solutions in order to treat cells with 5 µM to 100 µM final concentrations, using DMSO as control vehicle at the same dilution (0.1% DMSO for all conditions). The cells were treated 24 hours post-transfection with these drugs during 16 hours, except for the viability assays for which cells were treated during three days. After drug treatment, cells were used for immunoanalyses, cell viability assays or PC secretion assays.

Protein kinases and their activators or regulators (see Table 2) were expressed and purified, and their catalytic activity was assayed in the presence of a range of concentrations of each roscovitine analogue, as described previously (8, 9, 15). IC50 values were calculated from the dose-response curves.

### Immunoanalyses

Western blots and indirect immunofluorescence were performed as previously described (2, 12). For immunoblot analyses, total cell lysates were prepared in denaturing and reducing sample buffer (13), separated on 7.5% SDS-PAGE and transferred on nitrocellulose membranes using Trans-Blot system (Bio-Rad Laboratories, Hercules, CA). Saturated membranes were incubated with mouse monoclonal anti-ABCB4 (clone P3II-26; Enzo Life Sciences, Villeurbanne, France) or anti-α-tubulin (clone 1E4C11; ProteinTech, Manchester, United Kingdom) antibodies and then with peroxidase conjugated anti-mouse secondary antibodies (Sigma). Signals were detected with ECL prime western blotting detection reagent (GE Healthcare, Velizy-Villacoublay, France) and quantified in the linear range of detection using ImageJ 1.50i software (U.S. National Institutes of Health, Bethesda, MD).

For indirect immunofluorescence, HepG2 cells were grown on glass coverslips and after transfection and treatments, they were fixed and permeabilized during 1 min at -20°C in ice-cold methanol. Then ABCB4 and ABCC2 were immunolabelled using the mouse monoclonal P₃II-26 (IgG2b) and M₂I-4 (IgG1) antibodies (Enzo Life Sciences), respectively. AlexaFluor^{®} 555 and AlexaFluor^{®} 488 conjugated isotype-specific secondary antibodies (Thermo Fisher Scientific) and Hoechst 33342 (Thermo Fisher Scientific) were used to label ABCB4, ABCC2 and nuclei, respectively. Images were acquired using an IX83 inverted fluorescence microscope from Olympus (Rungis, France), equipped with a UPLSAPO 60XS2 silicone immersion objective and a Hamamatsu ORCA Flash4.0 digital CMOS camera, and analysed using Olympus CellSens Dimension Desktop version 1.16 and Adobe Photoshop version 8.0.1. For each experiment, all images were acquired with constant settings (acquisition time and correction of signal intensities).

### Cell viability assays

Cell viability was assessed by the conversion of MTT (3-[4,5-dimethylthiazol-2-yl]-2,5 diphenyl tetrazolium bromide) into formazan crystals by living cells, as described (14). In brief, HEK cells were seeded in 96-well plates in triplicate for each tested condition, including controls (no cells, no treatment, treatment with vehicle). After transient expression of ABCB4-WT as described above (to mimic the experimental conditions of the other experiments) and drug treatment during 72 hours, 125 µg/ml MTT (final concentration) was added in each well and cells were re-incubated at 37°C during 2 hours. These conditions were optimized in order to maintain the absorbance at 540 nm of the blanks below 0.1 OD unit (negative controls) and the absorbance at 540 nm for untreated cells between 1.0 and 1.3 OD units (positive controls). Then culture media were gently washed out, cells were lysed in 100 µL of pure DMSO and absorbance at 540 nm was measured using a multiplate cytofluorimeter SpectraFluor from Tecan (Männedorf, Switzerland). Cell viability was calculated for each triplicate after background subtraction and expressed as percentage of the mean for cells treated with vehicle only.

### Measurement of ABCB4-mediated phosphatidylcholine secretion

HEK cells were seeded in 0.01% poly-L-lysine (Sigma) pre-coated 6-well plates of 10 cm² area per well (1.3×10⁶ cells per well). After transient transfection of the empty vector (control), plasmids encoding ABCB4-WT or its variants, cells were incubated during 24 hours at 37°C in serum-free medium supplemented with 0.5 mM sodium taurocholate (Sigma) and 0.02% fatty acid-free bovine serum albumin (Sigma). Then, the secreted PC was quantified from the collected media using a fluoro-enzymatic assay previously described (12). Each condition was analysed in triplicate, after background subtraction, and results were normalized to the expression levels of ABCB4 determined by immunoblots of the corresponding cell lysates.

### Statistics

Graphics and non-parametric analyses of variance tests (Kruskall-Wallis) tests were performed using Prism version 7.00 (GraphPad Software, La Jolla, CA). A P value of less than 0.05 was considered significant. If not specified in figure legends, symbols indicate the comparison between the control (or vehicle-treated) and the other tested conditions.

### Example 3: Roscovitine rescues the maturation and the canalicular localisation of the I541F ER-retained ABCB4 variant

In this study, we used ABCB4-I541F, a prototypical ER-retained variant of ABCB4, first identified in a homozygous PFIC3 patient (1) and further characterized in our laboratory (3, 6). Indeed, ABCB4-I541F has been shown to be retained in the ER as an immature and high-mannose glycosylated protein (6), characterized by the absence or low abundance of a mature protein band on immunoblot, compared to the WT protein (Fig. 1A). We have previously demonstrated that the maturation and the localisation at the canalicular membrane of ABCB4-I541F could be partially rescued upon temperature shift of cell culture at 27°C or treatment with the ABCB1/MDR1 substrate CsA (3, 6). Since roscovitine at 100 µM was described to correct the function of the F508del ER-retained CFTR/ABCC7 variant (5), we investigated the potential correction of ABCB4-I541F by this molecule. Such treatment led to the correction of the maturation of ABCB4-I541F (Fig. 1A) in HEK cells, as shown by the quantification of immunoblots (Fig. 1B). These results were confirmed by indirect immunofluorescence analyses in HepG2 cells, a hepatocellular carcinoma derived cell line forming pseudo-bile canaliculi in cell culture (7). Indeed, the increased maturation of ABCB4-I541F was associated with its relocalisation at the canalicular membrane after treatment with 100 µM roscovitine in HepG2 cells (Fig. 1C). However, roscovitine displayed important dose-dependent cytotoxicity (Fig. 1D) and inhibition of ABCB4-WT-mediated phosphatidylcholine secretion activity in HEK cells (Fig. 1E), which may preclude its relevance for further investigations.

### Exemple 4: Structural analogues of roscovitine rescue the maturation and the canalicular localisation of ABCB4-I541F

The cytotoxic effect induced by roscovitine might be explained by its CDK inhibition activity(16). We thus synthesized structural analogues (Fig. 2A) potentially devoid of this feature. Aftin-4 is a methyl-roscovitine with reduced kinase inhibition activity (8, 9), and M3 is the main hepatic metabolite of roscovitine. All other products are carboxylated analogues displaying much reduced kinase inhibition compared to the parent compound, roscovitine (Table 2). This might indicate that carboxylation at the hydroxyl level and/or methylation at position N6 (Fig. 2A) strongly reduce CDK inhibition activity of these molecules. As expected, all roscovitine analogues with decreased CDK inhibition activity were less cytotoxic (Table 2, see below).

**TABLE 2. IC₅₀ of protein kinases after treatment with roscovitine and its structural analogues (µM)**

| **Kinases** | **CDK2/A*¹*** | **CDK5/p25*¹*** | **CDK9/T*¹*** | **CK1** | **CLK1** | **DYRK1A** | **GSK3** |
|---|---|---|---|---|---|---|---|
| Roscovitine | 0.080 | 0.210 | 0.533 | 4.3 | 2.9 | 3.33 | >10 |
| M3 | 9.0 | 20 | >33 | >33 | >33 | 19 | >10 |
| MRT2-163 | 6.0 | 20 | >33 | 20 | >33 | >10 | >10 |
| MRT2-164 | 2.2 | 10 | 17 | 28 | >33 | >10 | >10 |
| MRT2-235 | 5.9 | 8.3 | 7 | 7 | 11 | 21 | >10 |
| MRT2-237 | 3.4 | 7.7 | 9 | 3.33 | 8.5 | 13 | >10 |
| MRT2-239 | 8.0 | 11 | 20 | 4 | 9 | 21 | >10 |
| MRT2-241 | 5.1 | 17 | 8 | 11 | 13 | 20 | >10 |
| MRT2-243 | 3.0 | 6.3 | 5 | 6.1 | 20 | 12 | >10 |
| MRT2-245 | 6.3 | 15 | 28 | 17 | 13 | 21 | >10 |
| MRT2-249 | 9.0 | 27 | >33 | 5.0 | 23 | 9 | >10 |

| | | | | | | | |
|---|---|---|---|---|---|---|---|
| Abbreviations: CDK, cyclin-dependent kinase; CK1, casein kinase 1; CLK1, Cdc2-like kinase 1 (CLK1); DYRK1A, dual specificity tyrosine phosphorylation regulated kinase 1A; GSK3, glycogen synthase kinase 3B. *¹* The activators/regulators of CDKs are indicated after the slashes: A, cyclin A; p25; T, cyclin T. | | | | | | | |

Interestingly, in HEK cells, some of these analogues (MRT2-235, -237, -239, -241, -243, -245 and -249) were able to significantly increase the expression of a mature form of ABCB4-I541F while other analogues (Aftin-4, M3, MRT2-163 and MRT2-164) were not (Fig. 2B). The quantification of these results indicated a ~2.5-fold increase of expression of the mature form of ABCB4-I541F after treatment with 100 µM of the most potent roscovitine analogues (Fig. 2C). We then analysed the efficacy of the most potent analogues to correct the canalicular targeting of ABCB4-I541F in HepG2 cells.

After 16 hours of treatment with 100 µM of these compounds, we observed partial relocalisation of ABCB4-I541F at bile canaliculi, as shown by its partial co-localisation with ABCC2 (Fig. 3). Altogether, these results demonstrate that the maturation and the canalicular localisation of ABCB4-I541F can be rescued by new structural analogues of roscovitine.

### Example 5: Structural analogues of roscovitine are less cytotoxic and less inhibitory of ABCB4 activity than roscovitine

To pursue this study, we decided to focus on three roscovitine analogues (MRT2-235, MRT2-237 and MRT2-243) which displayed significant rescue of the maturation and partial relocalisation of ABCB4-I541F at bile canaliculi (Fig. 2B-C and Fig. 3). At concentrations lower than 100 µM, these analogues were much less cytotoxic than roscovitine in HEK cells (Fig. 4A). Indeed, the cell viability was still higher than 80% at 25 µM of the analogues while it was reduced to less than 60% at this concentration for roscovitine (Fig. 4A). Furthermore, measurement of ABCB4-WT-mediated PC secretion in HEK cells indicated that the three roscovitine analogues were less inhibitory than the original molecule when used at 10 µM and 25 µM (Fig. 4B). The IC₅₀ on ABCB4-WT activity was 7.5 µM for roscovitine while it was 2.5 to 4.0-fold higher for its analogues (Fig. 4C and Table 3).

**TABLE 3. IC₅₀ of roscovitine and analogues on the PC secretion activity of ABCB4-WT.**

| **Compounds** | **roscovitine** | **MRT2-235** | **MRT2-237** | **MRT2-243** |
|---|---|---|---|---|
| log IC₅₀ | -5.124 ± 0.078 | -4.678 ± 0.086 | -4.533 ± 0.092 | -4.754 ± 0.084 |
| IC50 (µM) | 7.51 | 20.97 | 29.31 | 17.63 |
| *(min-max)* | *(5.49-10.28)* | *(14.87-29.62)* | *(20.30-42.32)* | *(12.60-24.64)* |

| | | | | |
|---|---|---|---|---|
| IC₅₀ were calculated from dose-response curves shown in Fig. 4C. | | | | |

It is also important to notice that treatment with roscovitine and these three analogues at 10, 25 and 100 µM do not significantly alter the plasma membrane localization of ABCB4-WT in HEK cells (Fig. 7). Thus, inhibition of ABCB4-mediated PC secretion by roscovitine and its analogues may be due to a direct effect of these compounds with ABCB4 function. Altogether, these results indicate that roscovitine analogues might be interesting ABCB4 correctors with low cytotoxicity and low inhibition activity of ABCB4.

### Example 6: Roscovitine analogues rescue the maturation, the localisation and the activity of ABCB4-I541F

The inhibition of ABCB4 activity induced by MRT2-235, MRT2-237 and MRT2-243 when used at 100 µM led us to investigate these analogues at lower concentrations. After treatment with 5, 10 and 25 µM of the three analogues, we observed a dose-dependent correction of ABCB4-I541F maturation in HEK cells (Fig. 5A; quantification in Fig. 5B). Treatment with 25µM of these analogues triggered partial relocalisation of ABCB4-I541F at bile canaliculi in HepG2 cells (Fig. 5C). Finally, we measured the PC secretion activity of ABCB4-I541F in HEK cells after treatment with or without roscovitine analogues at 25 µM. Importantly, we noticed a marked correction of ABCB4-I541F activity from 7.1 ± 0.8% of residual activity with control treatment (vehicle) to 19.0 ± 3.4%, 13.0 ± 3.5% and 22.5 ± 6.2% after treatment with 25 µM of MRT2-235, MRT2-237 and MRT2-243, respectively (Fig. 5D). Altogether, our results demonstrate that the three selected roscovitine analogues are able to retrieve the maturation and the canalicular localisation of the ER-retained ABCB4-I541F variant and to significantly correct its PC secretion activity.

### Example 7: Roscovitine analogues rescue the function of two other ER-retained ABCB4 variants

To extend this study, we analysed the effect of roscovitine analogues on two other ER-retained missense ABCB4 variants, I490T and L556R, identified in patients with liver cancer (10) or PFIC3(1). Without treatment, these variants were mainly detected as immature proteins by immunoblot in HEK cells (Fig. 6A-B). However, after treatment with 5 to 25 µM of the three roscovitine analogues, we observed a dose-dependent rescue of the maturation of these two variants (Fig. 6A-B), as confirmed by the quantification of these experiments (Fig. 6C-D). These results were further validated by indirect immunofluorescence assays in HepG2 cells: both I490T and L556R variants were partially relocalised at bile canaliculi upon treatment with 25 µM of MRT2-235, MRT2-237 or MRT2-243 (Fig. 6E-F). As expected, the PC secretion activity of these two ER-retained variants in HEK cells was strongly impaired in vehicle-treated cells (Fig. 6G-H). However, after treatment with 25 µM of the three roscovitine analogues, we observed a significant increase of the residual activity of these variants (Fig. 6G-H). These results provide evidence that roscovitine analogues are able to rescue the localisation, the maturation and the PC secretion activity of several ER-retained ABCB4 variants.

### References

(1) Jacquemin, E. et al. The wide spectrum of multidrug resistance 3 deficiency: from neonatal cholestasis to cirrhosis of adulthood. Gastroenterology 120, 1448-1458. (2001).
(2) Delaunay, J. L. et al. A functional classification of ABCB4 variations causing progressive familial intrahepatic cholestasis type 3. Hepatology 63, 1620-1631 (2016).
(3) Gautherot, J. et al. Effects of Cellular, chemical and pharmacological chaperones on the rescue of a trafficking-defective mutant of the ATP-binding cassette transporters ABCB1/ABCB4. The Journal of biological chemistry 287, 5070-5078 (2012).
(4) Meijer, L. et al. Biochemical and cellular effects of roscovitine, a potent and selective inhibitor of the cyclin-dependent kinases cdc2, cdk2 and cdk5. European journal of biochemistry 243, 527-536 (1997)
(5) Norez, C. et al. Roscovitine is a proteostasis regulator that corrects the trafficking defect of F508del-CFTR by a CDK-independent mechanism. British journal of pharmacology 171, 4831-4849 (2014).
(6) Delaunay, J. L. et al. A missense mutation in ABCB4 gene involved in progressive familial intrahepatic cholestasis type 3 leads to a folding defect that can be rescued by low temperature. Hepatology 49, 1218-1227 (2009).
(7) Sormunen, R., Eskelinen, S. & Lehto, V. P. Bile canaliculus formation in cultured HEPG2 cells. Laboratory investigation; a journal of technical methods and pathology 68, 652-662 (1993).
(8) Tang, L. et al. Crystal structure of pyridoxal kinase in complex with roscovitine and derivatives. The Journal of biological chemistry 280, 31220-31229 (2005).
(9) Bettayeb, K. et al. Small-molecule inducers of Abeta-42 peptide production share a common mechanism of action. FASEB Journal. 26, 5115-5123 (2012)
(10) Tougeron, D., Fotsing, G., Barbu, V. & Beauchant, M. ABCB4/ MDR3 gene mutations and Cholangiocarcinomas. J Hepatol 57, 467-468 (2012).
(11) Andress, E. J., Nicolaou, M., McGeoghan, F. & Linton, K. J. ABCB4 missense mutations D243A, K435T, G535D, I490T, R545C, and S978P significantly impair the lipid floppase and likely predispose to secondary pathologies in the human population. Cellular and molecular life sciences 74, 2513-2524 (2017).
(12) Gautherot, J. et al. Phosphorylation of ABCB4 impacts its function: Insights from disease-causing mutations. Hepatology 60, 610-621 (2014).
(13) Laemmli, U. K. Cleavage of structural proteins during the assembly of the head of bacteriophage T4. Nature 227, 680-685 (1970).
(14) van Meerloo, J., Kaspers, G. J. & Cloos, J. Cell sensitivity assays: the MTT assay. Methods in molecular biology (Clifton, N.J.) 731, 237-245 (2011).
(15) Bach, S. et al. Roscovitine targets, protein kinases and pyridoxal kinase. The Journal of biological chemistry 280, 31208-31219 (2005).
(16) Meijer, L. et al. Biochemical and cellular effects of roscovitine, a potent and selective inhibitor of the cyclin-dependent kinases cdc2, cdk2 and cdk5. European journal of biochemistry 243, 527-536 (1997).

## Claims

1. Compound of formula (I): 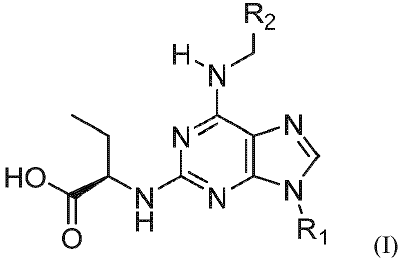 wherein
R₁ is
- a (C₁-C₄) alkyl group, or
- a (C₃-C₆) cycloalkyl group,
R₂ is
- a phenyl or a substituted phenyl with one to three groups,
with the proviso that when R₂ is a phenyl then R₁ is a (C₃-C₆) cycloalkyl group,
or anyone of its pharmaceutically acceptable salt.

2. Compound according to claim 1, wherein R₁ is a (C₁-C₄) alkyl group selected from methyl, ethyl, propyl, isopropyl, preferably is isopropyl.

3. Compound according to claim wherein R₁ is cyclopentyl.

4. Compound according to claim 1, wherein R₂ is a phenyl or a substituted phenyl with one or two groups, said groups being selected from electron withdrawing groups and halogens, preferably R₂ is a phenyl or a substituted phenyl with one or two groups, said groups being selected from CF₃, F, Cl and Br.

5. Compound according to any one of claims **1** or **4,** wherein R₂ is a substituted phenyl with one to three groups and at least one group being in *meta* position, preferably one or two groups and at least one group being in *meta* position.

6. Compound according to any one of claims **1** to **5,** wherein R₁ is a cyclopentyl group and the group R₂ is selected from:

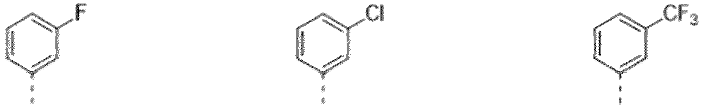

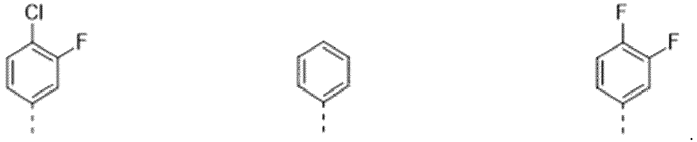

7. Compound according to any one of claims **1** to **6,** wherein said compound is selected from:

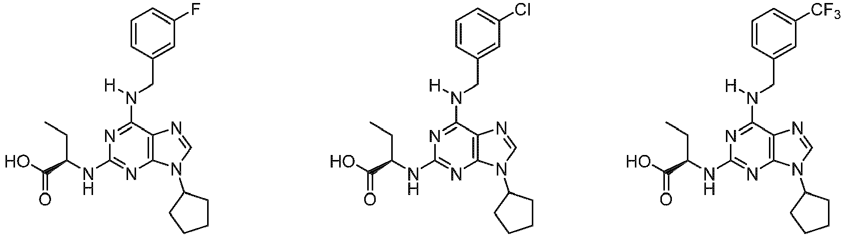

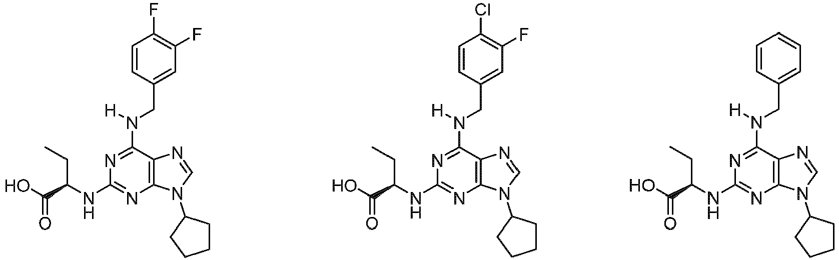

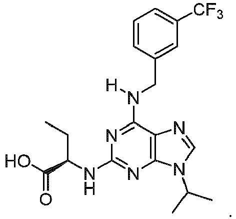

8. A composition comprising at least one compound according to any one of claims **1** to **7.**

9. The composition according to claim **8,** wherein said composition is a pharmaceutical composition further comprising at least one pharmaceutically acceptable excipient.

10. Compound according to any one of claims **1** to **7** for use as a medicament.

11. A composition according to claim **8** or **9** for use as a medicament.

12. The compound for use according to any one of claims **1** to **7** or the composition for use according to claim **8** or **9** for use in the treatment of rare biliary diseases such as intrahepatic cholestatic diseases.

13. The compound for use or the composition for use according to claim **12,** wherein the intrahepatic cholestatic diseases is an ABCB4-related biliary disease, **characterized by** a defect of phosphatidylcholine secretion in hepatocytes.

14. The compound for use or the composition for use according to claim **12** or **13,** wherein the biliary disease is chosen from intrahepatic cholestatic diseases, progressive familial intrahepatic cholestasis (PFIC3), low phospholipid-associated cholelithiasis (LPAC) syndrome, intrahepatic cholestasis of pregnancy (ICP), drug-induced liver injury, transient neonatal cholestasis (TNC), adult biliary fibrosis and cirrhosis or intrahepatic cholangiocarcinoma (IHCC).

15. The compound for use or the composition for use according to any one of claims **12** to **14,** wherein intrahepatic cholestatic diseases are selected from the group consisting of: progressive familial intrahepatic cholestasis type 3, LPAC syndrome or ICP.

## Patentansprüche

1. Verbindung der Formel (I): 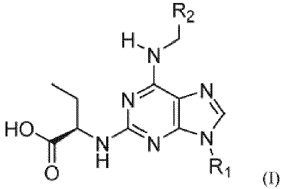 wobei
R₁
- eine (C₁-C₄)-Alkylgruppe, oder
- eine (C₃-C₆)-Cycloalkylgruppe ist,
R₂
- ein Phenyl oder ein substituiertes Phenyl mit einer bis drei Gruppen ist,
mit der Maßgabe, dass wenn R₂ ein Phenyl ist, R₁ eine (C₃-C₆)-Cycloalkylgruppe ist,
oder eines ihrer pharmazeutisch akzeptablen Salze.

2. Verbindung nach Anspruch **1,** wobei R₁ eine (C₁-C₄)-Alkylgruppe ist, ausgewählt aus Methyl, Ethyl, Propyl, Isopropyl, vorzugsweise Isopropyl ist.

3. Verbindung nach Anspruch **1,** wobei R₁ Cyclopentyl ist.

4. Verbindung nach Anspruch **1,** wobei R₂ ein Phenyl oder ein substituiertes Phenyl mit einer oder zwei Gruppen ist, wobei die Gruppen ausgewählt sind aus elektronenziehenden Gruppen und Halogenen, vorzugsweise R₂ ein Phenyl oder ein substituiertes Phenyl mit einer oder zwei Gruppen ist, wobei die Gruppen aus CF₃, F, Cl und Br ausgewählt sind.

5. Verbindung nach einem der Ansprüche **1** oder **4,** wobei R₂ ein substituiertes Phenyl mit einer bis drei Gruppen ist, und sich mindestens eine Gruppe in *meta-*Position befindet, vorzugsweise eine oder zwei Gruppen, und sich mindestens eine Gruppe in *meta*-Position befindet.

6. Verbindung nach einem der Ansprüche **1** bis **5,** wobei R₁ eine Cyclopentylgruppe ist, und die Gruppe R₂ ausgewählt ist aus:

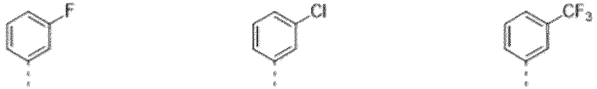

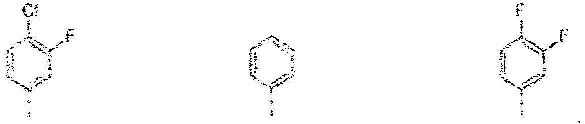

7. Verbindung nach einem der Ansprüche **1** bis **6,** wobei die Verbindung ausgewählt ist aus:

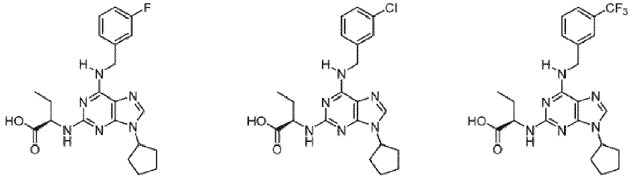

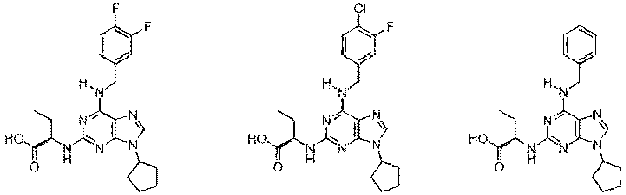

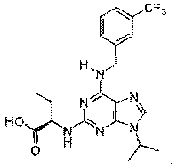

8. Zusammensetzung, die mindestens eine Verbindung nach einem der Ansprüche **1** bis **7** umfasst.

9. Zusammensetzung nach Anspruch **8,** wobei es sich bei der Zusammensetzung um eine pharmazeutische Zusammensetzung handelt, die weiter mindestens einen pharmazeutisch akzeptablen Hilfsstoff umfasst.

10. Verbindung nach einem der Ansprüche **1** bis **7** zur Verwendung als Medikament.

11. Zusammensetzung nach Anspruch **8** oder **9** zur Verwendung als Medikament.

12. Verbindung zur Verwendung nach einem der Ansprüche **1** bis **7** oder Zusammensetzung zur Verwendung nach Anspruch **8** oder **9** zur Verwendung in der Behandlung seltener biliärer Erkrankungen, wie etwa intrahepatischer cholestatischer Erkrankungen.

13. Verbindung zur Verwendung oder Zusammensetzung zur Verwendung nach Anspruch **12,** wobei es sich bei den intrahepatischen cholestatischen Erkrankungen um eine ABCB4-bezogene biliäre Erkrankung handelt, die durch einen Defekt der Phosphatidylcholin-Sekretion in Hepatozyten gekennzeichnet ist.

14. Verbindung zur Verwendung oder Zusammensetzung zur Verwendung nach Anspruch **12** oder **13,** wobei die biliäre Erkrankung ausgewählt ist aus intrahepatischen cholestatischen Erkrankungen, progressiver familiärer intrahepatischer Cholestase (PFIC3), Syndrom der mit niedrigem Phospholipidspiegel assoziierten Cholelithiasis (LPAC), intrahepatischer Schwangerschaftscholestase (ICP), arzneimittelbedingten Leberschäden, transienter neonataler Cholestase (TNC), adulter biliärer Fibrose und Zirrhose oder intrahepatischem Cholangiokarzinom (IHCC).

15. Verbindung zur Verwendung oder Zusammensetzung zur Verwendung nach einem der Ansprüche **12** bis **14,** wobei intrahepatische cholestatische Erkrankungen ausgewählt sind aus der Gruppe bestehend aus: progressiver familiärer intrahepatischer Cholestase Typ 3, LPAC-Syndrom oder ICP.

## Revendications

1. Composé de formule (I) : 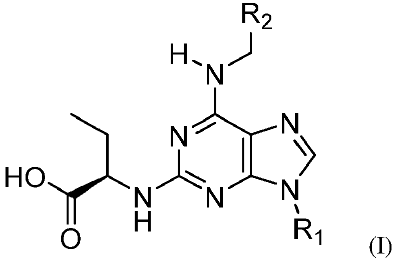 dans laquelle
R₁ est
- un groupe alkyle en (C₁-C₄), ou
- un groupe cycloalkyle en (C₃-C₆),
R₂ est
- un phényle ou un phényle substitué par un à trois groupes,
à condition que, lorsque R₂ est un phényle, R₁ est un groupe cycloalkyle en (C₃-C₆),
ou l'un de ses sels pharmaceutiquement acceptables.

2. Composé selon la revendication **1,** dans lequel R₁ est un groupe alkyle en (C₁-C₄) choisi parmi les groupes méthyle, éthyle, propyle, isopropyle, de préférence isopropyle.

3. Composé selon la revendication **1,** dans lequel R₁ est un cyclopentyle.

4. Composé selon la revendication **1,** dans lequel R₂ est un phényle ou un phényle substitué avec un ou deux groupes, lesdits groupes étant choisis parmi les groupes électroattracteurs et les halogènes, de préférence R₂ est un phényle ou un phényle substitué avec un ou deux groupes, lesdits groupes étant choisis parmi CF₃, F, Cl et Br.

5. Composé selon l'une quelconque des revendications **1** ou **4,** dans lequel R₂ est un phényle substitué avec un à trois groupes et au moins un groupe étant en position *méta,* de préférence un ou deux groupes et au moins un groupe étant en position *méta.*

6. Composé selon l'une quelconque des revendications **1** à **5,** dans lequel R₁ est un groupe cyclopentyle et le groupe R₂ est choisi parmi :

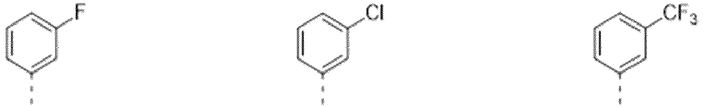

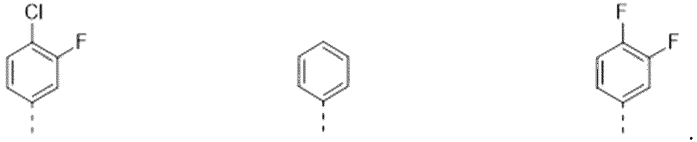

7. Composé selon l'une quelconque des revendications **1** à **6,** dans lequel ledit composé est choisi parmi :

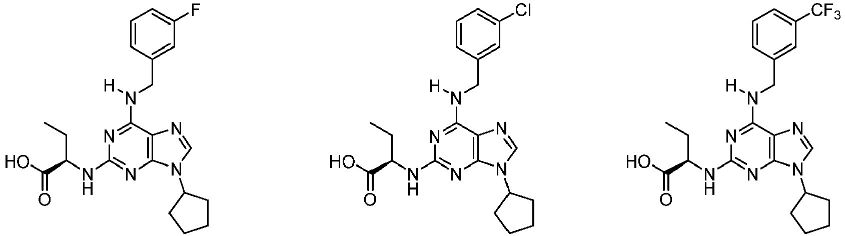

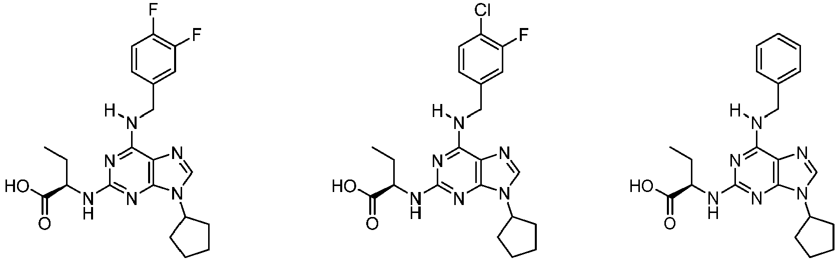

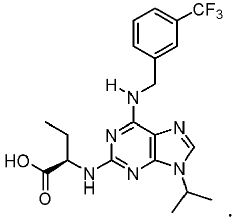

8. Composition comprenant au moins un composé selon l'une quelconque des revendications **1** à **7.**

9. Composition selon la revendication **8,** dans laquelle ladite composition est une composition pharmaceutique comprenant en outre au moins un excipient pharmaceutiquement acceptable.

10. Composé selon l'une quelconque des revendications **1** à **7** pour utilisation en tant que médicament.

11. Composition selon la revendication **8** ou **9** pour utilisation en tant que médicament.

12. Composé pour utilisation selon l'une quelconque des revendications **1** à **7** ou composition pour utilisation selon la revendication **8** ou **9** pour utilisation dans le traitement de maladies biliaires rares telles que les maladies cholestatiques intrahépatiques.

13. Composé pour utilisation ou composition pour utilisation selon la revendication **12,** dans lequel les maladies cholestatiques intrahépatiques sont des maladies biliaires liées à l'ABCB4, **caractérisées par** un défaut de sécrétion de phosphatidylcholine dans les hépatocytes.

14. Composé pour utilisation ou composition pour utilisation selon la revendication **12** ou **13,** dans lequel la maladie biliaire est choisie parmi les maladies cholestatiques intrahépatiques, la cholestase intrahépatique familiale progressive (PFIC3), le syndrome de cholélithiase associée à une faible teneur en phospholipides (LPAC), la cholestase intrahépatique de la grossesse (ICP), les lésions hépatiques induites par des médicaments, la cholestase néonatale transitoire (CNT), la fibrose biliaire et la cirrhose de l'adulte ou le cholangiocarcinome intrahépatique (IHCC).

15. Composé pour utilisation ou composition pour utilisation selon l'une quelconque des revendications **12** à **14,** dans lequel les maladies cholestatiques intrahépatiques sont choisies dans le groupe constitué de : cholestase intrahépatique familiale progressive de type 3, syndrome LPAC ou ICP.
